# EUROPEAN PATENT SPECIFICATION

(11) **EP 1 173 205 B1**
(45) Date of publication and mention of the grant of the patent: **01.06.2005**
(21) Application number: 00928046.2
(22) Date of filing: 19.04.2000
(51) Int. Cl.: A61K 45/00, A61K 9/20, A61P 3/06

(54) **AN ORAL FORMULATION FOR ILEUM ADMINISTERING COMPRISING AN INHIBITOR COMPOUND OF THE ILEAL BILE ACID TRANSPORT**
ORALE FORMULIERUNG ZUR VERABREICHUNG IN DEN ILEUM ENTHALTEND EINEN HEMMSTOFF DES ILEUM-GALLENSÄURETRANSPORTS
FORMULATION ORALE POUR ADMINISTRATION ILEALE RENFERMANT UN COMPOSE INHIBITEUR DU TRANSPORT DES ACIDES BILIAIRES PRESENTS DANS L'ILEON

(30) Priority: 19.04.1999 SE 9901387
(43) Date of publication of application: 23.01.2002
(73) Proprietor: AstraZeneca AB, 151 85 Södertälje (SE)
(72) Inventor: ABRAHAMSSON, Bertil, S-431 83 Mölndal (SE); LINDQVIST, Ann-Margret, S-431 83 Mölndal (SE); UNGELL, Anna-Lena, S-431 83 Mölndal (SE)
(74) Representative: Bill, Kevin
(86) International application number: PCT/SE2000/000755
(87) International publication number: WO 2000/062810

(56) References cited:
- US-A- 5 614 220
- US-A- 5 723 458
- DATABASE EMBASE DIALOG INFORMATION SERVICES, FILE 73 ACC. NO. 02888148, EMBASE NO. 1985132107 JACOBSEN O. ET AL.: 'Effect of entercoated cholestyramine on bowel habit after ileal resection: A double blind crossover study', XP002907347 & BR. MED. J. vol. 290, no. 6478, 1985, (UNITED KINGDOMS), pages 1315 - 1318

## Description

### FIELD OF THE INVENTION

The present invention relates to an oral pharmaceutical dosage form comprising a substance with inhibiting effect on the ileal bile acid transport system (IBAT) and a bile acid binder, wherein the formulation is designed to deliver the bile acid binder in the colon. More specifically, the dosage form is suitable in the treatment of hypercholesterolaenua. The invention also relates to manufacturing processes and the use of the dosage form in the treatment of hypercholesterolaemia. A further aspect of the invention is the use of a substance with inhibiting effect on IBAT in combination with a bile acid binder by simultaneously, separately or sequentially administration of the two substances, and the use of these substances in the manufacture of such a pharmaceutical dosage form.

### BACKGROUND OF THE INVENTION AND PRIOR ART

It is well known that hyperlipidemic conditions associated with elevated concentrations of total cholesterol and low-density lipoprotein cholesterol are major risk factors for coronary heart disease and particularly artherosclerosis. Interfering with the circulation of bile acids within the lumen of the intestinal tracts is found to reduce the level of cholesterol. Previous established therapies to reduce the concentration of cholesterol involve for instance treatment with HMG-CoA reductase inhibitors, preferably statins such as simvastin and fluvastin, or treatment with bile acid binders, such as resins. Frequently used bile acid binders are for instance cholestyramine and cholestipol. One recently proposed therapy involves the treatment with substances with inhibiting effect on the ileal bile acid transport system (IBAT).

Re-absorption of bile acid from the gastro-intestinal tract is a normal physiological process, which mainly takes place in the ileum by an active transport mechanism called ileal bile acid transport (IBAT). Inhibitors of IBAT can be used in the treatment of hypercholesterolaemia. See for instance "Interaction of bile acids and cholesterol with nonsystemic agents having hypocholesterolemic properties", Biochemica et Biophysica Acta, 1210 (1994) 255- 287. Thus, suitable compounds having such inhibitory IBAT activity are also useful in the treatment of hyperlipidaemic conditions.

Several chemical compounds possessing such IBAT activity have recently been described, see for instance hypolipidaemic benzothiazepine compounds described in International Patent Application, Publication No. WO 93/16055 and WO 96/16051; condensed 1,4-thiazepines described in International Patent Application, Publication No. WO 94/18183; different heterocyclic compounds described in International Patent Application, Publication No. WO 94/18184; and 1,4-benzothiazepine-1,1-dioxides described in International Patent Application, Publication No. WO 96/05188, all of which are hereby incorporated by reference.

Further, especially suitable compounds for the present invention are for instance benzothiazepines with IBAT activity described in International Patent Application, Publication No. WO 96/08484; bile acid resorption inhibitors described in International Patent Application, Publication No. WO 97/33882, WO 98/07449 and WO 98/03818, and in European Patent Application, Publication No. EP-A-0864582, EP-A-0489423, EP-A-0549967, EP-A-0573848, EP-A-0624593, EP-A-0624594, EP-A-0624595, and EP-A-0624596, all of which are hereby incorporated by reference. Further compounds of interest can be found in International Patent Application, Publication No. WO 99/32478, WO 99/64409 and WO 00/01687, all of which are hereby incorporated by reference.

It is proposed that these types of compounds can be administered by any conventional means available for use in conjunction with pharmaceuticals. For instance, the dosage forms can be a daily dose which is administered once a day or being divided to be administered several times a day, or alternative in a sustained release form. Suitable dosage forms are intended for oral administration.

All benzothiazepines, however, will not be effective as IBAT inhibitor compounds. Thus, diltiazem, which is a 1,5-benzothaizepine, is a calcium blocker with coronary vasodilating activity (see The Merck Index, Merck & Co, Inc., 12th ed., 1996, p. 541). With respect to inhibition of IBAT, diltiazem has no activity.

In general, pharmaceutical drug substances will be absorbed in the upper small intestine, and therefore only a small amount will reach ileum when administered in a conventional oral dosage form. Irrespective of the construction of the pharmaceutical dosage form, it should provide contact for the active compound, e.g. inhibitor of IBAT, with the compound's site of action in the body, for example in the ileum. The above prior art documents discuss in general terms suitable pharmaceutical dosage forms for the described IBAT inhibitor compounds. However, none of the documents describe a specific way to obtain a release of the active substance directly to or close to the site of action.

Contact between the active drug and the site of action can be established in different ways.

It has been proposed that after absorption over the gastro-intestinal membrane, an IBAT inhibitor could interact with transport systems similar to IBAT for instance the corresponding transport system in the liver (LBAT) or could provide other non-specific systemic effects which could lead to undesirable pharmacological or even toxicological effects. This could severely limit the clinical usefulness of IBAT inhibitors especially in the treatment of hypercholesterolaemia, i.e. conditions associated with elevated concentra-tions of total cholesterol and low-density lipoprotein cholesterol.

The inhibition of the re-absorption of bile acids from the small intestine performed by an effective IBAT inhibitor may lead to increased levels of bile acids in the lower parts (colon) of the gastro-intestinal tract. Such an increase of bile acid concentrations in the distal regions could potentially generate diarrhoea and discomfort to the patient. The present invention provides a new approach to minimise the concentration of free bile acids in the colon and thereby reduce the potential risk of adverse events by co-administration of a bile acid binder together with the IBAT inhibitor. However, the combination of an IBAT inhibitor and a bile acid binder have previously been proposed in the above patent applications describing new IBAT inhibitor compounds. The purpose of such previously described combinations have been to enhance the cholesterol lowering efficacy of the therapy, and there is no hint that such a combination could be used to minimise a potential risk for diarrhoea connected with IBAT inhibitor therapy.

### BRIEF DESCRIPTION OF THE INVENTION

The aim of the present invention is to reduce the problem with undesirable side effects of IBAT inhibitor compounds.

Preferably, the formulation is an orally administered formulation, such as a delayed release formulation, which starts to release the main part of the drug in the distal jejunum or in the proximal ileum. The oral formulation might also provide protection of the drug from the acid environment in the stomach by an enteric coating. Such an enteric coating also protects the gastric mucosa from drug exposure and thereby minimises irritation or even damages of the gastric mucosa potentially caused by aggressive drug exposure.

The aim of the present invention is to provide a combination for simultaneous, separate or sequential administration which combination comprises an IBAT inhibitor and a bile acid binder. Such a combination will protect the patient from any possible side effect caused by excess of bile acids in the colon, such as diarrhoea. If the transport of bile acids is blocked by an IBAT inhibitor the bile acids might be deposited in the colon and induce a secretory diarrhoea - by irritation and inflammation - as a undesired side effect caused by the treatment with an IBAT inhibitor.

In the provided combination therapy the bile acid binder, for instance a resin such as cholestyramine or cholestipol, is administered in a dosage form with colon release of the bile acid binder. A colon release formulation.will provide protection of the bile acid binder to the luminal contents in the more proximal parts of the intestine, where the bile acid concentrations are high. Such a formulation will prevent binding of bile acids to the bile acid binder before the formulation reaches the colon. Thereby, maximal bile acid binding capacity will be obtained in the colon and any possible gastro-intestinal side effects, such as diarrhoea, may be avoided. Thus, any additional amount of bile acid presented in the colon due to the treatment with the IBAT inhibitor compound, would be bound to a bile acid binder, which the bile acid binder is preferably delivered in the colon, thereby any possible side effects such as diarrhoea is avoided.

### DETAILED DESCRIPTION OF THE INVENTION

### IBAT inhibitor compounds

Active ingredients suitable as IBAT inhibitor compounds in the present invention are those exhibiting activity when screening for IBAT inhibiting properties. Suitable examples of such compounds can be found in the references cited on page 2 of the present application.

Active ingredients particularly suitable as IBAT inhibitor compounds in the present invention include benzothiazepines, and more particularly 1,4-benzothiazepines and 1,5-benzothiazepines exhibiting activity when screening for IBAT inhibiting properties. Of these, compounds with an oxidized sulfur group, particularly a sulfone group, in the 7 membered ring are preferred. Furthermore, the presence of an amine group in the 7 membered ring is preferred.

### Pharmaceutical formulations

Dosage forms with a controlled lagtime can be constructed in different ways for instance as described in the following.

A controlled lagtime can be triggered by pH changes, redox potential differences or luminal metabolic changes in the gastro-intestinal tract as described in Aliment Pharmacol Ther 1997, 11 (suppl 3): 109-115. Such a controlled lagtime could be obtained for instance by a programmed disintegration of the formulation due to erosion, dissolution or in general by components present in the formulation interacting with the environment in the gastro-intestinal tract. Preferably, the drug release from the dosage form could be triggered by the pH variation between jejunum and ileum.

Alternatively, the drug release from the dosage form can be chronographic controlled to obtain the above specified time limits, such as for instance described in the European Patent Application, Publication No. EP-A-0384642.

According to another aspect of the invention, a sustained release formulation can be constructed by any known principle, such as eroding or non-eroding matrices, membrane-coating layers or by diffusion or osmotically driven drug release. Suitable techniques for the construction of such formulations are for instance described in M. E. Aulton, Pharmaceutics, The science of dosage form design. (1988).

In the present invention an IBAT inhibitor compound is combined with a bile acid binder thereby avoiding a possible risk of excess of bile acids in colon caused by the inhibition of the ileal bile acid transport system. An excess of bile acids in the visceral contents may cause diarrhoea. Thus, the present invention also provides a treatment of a possible side effect such as diarrhoea in patients during therapy comprising IBAT inhibitor compounds.

Suitable bile acid binders for such a combination therapy are resins, such as cholestyrmine and cholestipol. One advantage is that the dose of bile acid binder might be kept lower than the therapeutical dose for treatment of cholesterolemia in single treatment comprising solely a bile acid binder. By a low dose of bile acid binder any possible side effects caused by poor tolerance of the patient to the therapeutic dose could also be avoided.

The bile acid binder is administered in a dosage form with colon release, i.e. delivery of the active dose of bile acid binder in the colon. A possible risk of receiving an excess of bile acid in the colon by treatment with an IBAT inhibitor could be avoided by co-administration of a bile acid binder with colon release. Thus, any excess of bile acid in the colon, with a possible risk to cause diarrhoea, will be bound into a resin. The dose of the bile acid binder could be kept low due to an effective use of the dose by such a colon release. The colon delivery of the bile acid binder can be obtained by a formulation comprising a core containing the bile acid binder and optionally pharmaceutically acceptable excipients, and a coating of said core with a delayed release membrane adapted for colonic delivery. Technologies to obtain such a delivery of drugs to the colon are for example described in Drug Development and Industrial Pharmacy 1997, 23: 893-913.

Further general aspects of the invention are that the formulations can be solid, semi-solid or liquid formulations. In a solid formulation, the carrier can be monolithic, such as tablets or capsules. One preferred monolithic formulation is a coated tablet, a capsule comprising small, coated units or a multiple unit tablet comprising a multitude of small coated units. Semi-solid or liquid formulations can be administered in capsules suitable for such vehicles. The most preferred formulation is an oral formulation such as a tablet or a capsule comprising coated small units or pellets. The formulation or dosage form may contain from 0.05% to 95% of the active compound in admixture with a pharmaceutically acceptable carrier, or pharmaceutically acceptable excipients.

### Preparation of core material

The core material for the units, i.e. the tablets or the individual pellets can be constituted according to different principles. The core material may be homogenous or heterogeneous. The core containing the active principle may be differently formulated such as monolithic tablets, capsules, granules, pellets, other particles or crystals.

With a homogenous core material is meant, that it has a homogenous distribution of active substance throughout the core material.

The active substance, i.e. the IBAT inhibitor compound, is optionally mixed with further components to obtain preferred handling and processing properties and a suitable concentration of the active substance in the final mixture. Such components can be binders, surfactants, lubricants, glidants, fillers, additives or other pharmaceutically acceptable ingredients, alone or in mixtures.

Said core material may be produced either by direct compression of the mixed ingredients, or by granulation of the ingredients followed by compression of the granulated material.

In direct compression, the ingredients are mixed and compressed by using ordinary tableting equipment.

For the granulation there are numerous alternatives of granulating procedures mentioned in the literature, dry methods like roller compaction (Chilsonator) and wet methods utilizing granulating solutions with and without the addition of binders. A variant of the wet methods is to make a spray-granulation in a fluid bed.

For the wet granulating methods, either organic solvents, aqueous solutions or pure water may be utilized to prepare the granulating solutions. Due to environmental considerations pure water is preferred, if it is possible due to the composition of the mixture.

Homogenous core particles can also be prepared by techniques such as dry or wet milling, freeze milling, air-jet micronisation, spray drying, spray chilling, controlled crystallisation, supercritical crystallisation, emulsion solvent evaporation and emulsion solvent extraction.

The core material may also be produced by extrusion/spheronization, balling or compression, utilizing different process equipments.

The size of the formulated core materials is approximately between 2 and 14 mm, preferably between 3 and 9 mm for a tablet preparation, and between 0.001 and 4 mm, preferably between 0.001 and 2 mm for a pellet preparation.

The manufactured core material may be further layered with additional ingredients comprising the active substance and/or be used for further processing.

Alternatively, the core material may be heterogeneous with an inner zone, for instance a seed or sphere, not containing the active substance. A layer comprising the active substance, and optionally pharmaceutically acceptable excipients, surrounds this seed or sphere.

The seed or sphere may be soluble or insoluble. Optionally, the seed or sphere (inner zone) may be coated with an inert layer to prepare a smooth surface before the layer containing active substance is applied onto the seed/sphere.

Insoluble seeds/spheres may comprise different oxides, celluloses, organic polymers and other materials, alone or in mixtures. Water-soluble seeds/spheres may comprise different inorganic salts, sugars and other materials, alone or in mixtures. The size of the seeds may vary between approximately 0.1 and 2 mm. The seeds layered with the matrix containing the active substance are produced either by powder or solution/suspension layering using for instance granulating or spray coating/layering equipment.

### Processes for application ofdelayed release membranes

Delayed release membrane can be applied to the core material, being a monolithic tablet, multiple units or a hard or soft gelatine capsule, by coating or layering procedures in suitable equipment such as coating pans, coating granulators or in a fluidized bed apparatus using water and/or organic solvents for the coating process. Also powder-coating principles may be applied. Another possibility is to apply the coating by microencapsulation techniques such as coacervation, emulisification with subsequent removal of the solvent by extraction or evaporation, ionotropic gelation or congealing.

Such delayed release membranes may be applied on core material comprising the IBAT inhibitor for delivery to the distal small intestine and optionally also be applied to the bile acid binder for delivery to the colon.

### Pharmaceutical additives

Delayed release coatings may be obtained by one or more, separetely or in compatible combinations of pharmaceutically acceptable ingredients, in amounts carefully titrated to reach the intended release properties. As coating layer, the following pH sensitive polymers can be applied; e.g. methacrylic acid copolymers, cellulose acetate phthalate, hydroxypropyl methylcellulose phthalate, hydroxypropyl methylcellulose acetate succinate, polyvinyl acetate phthalate, cellulose acetate trimellitate, carboxymethyl ethylcellulose, shellac or other suitable enteric coating layer polymer(s). The coating layer may also be composed of film-forming polymers being sensitive to other luminal components than pH, such as bacterial degradation or a component that has such a sensitivity when it is mixed with another film-forming polymer. Examples of such components providing delayed release to the intended regions are; polymers comprising azo bond(s), polysaccharides such as pectin and its salts, galactomannans, amylose and chondroitin, disulphide polymers and glycosides.

The delayed release coating or an additional coating of the formulation may contain other film-forming polymers being non-sensitive to the luminal conditions for technical reasons or chronographic control of the drug release. Materials to be used for such purpose includes, but are not limited to; sugar, polyethylene glycol, polyvinylpyrrolidone, polyvinyl alcohol, polyvinyl acetate, hydroxypropyl cellulose, methylcellulose, ethylcellulose, hydroxypropyl methylcellulose, carboxymethylcellulose sodium and others, used alone or in mixtures.

Additives such as dispersants, colorants, pigments, additional polymers e.g. poly(ethylacrylat, methylmethacrylat), anti-tacking and anti-foaming agents may also be included into the coating layer. Other compounds may be added to increase film thickness and to decrease diffusion of acidic gastric juices into the core material.

The coating layers may also contain pharmaceutically acceptable plasticizers to obtain desired mechanical properties. Such plasticizers are for instance, but not restricted to, triacetin, citric acid esters, phthalic acid esters, dibutyl sebacate, cetyl alcohol, polyethylene glycols, glycerol monoesters, polysorbates or other plasticizers and mixtures thereof. The amount of plasticizer is preferably optimised for each formula, in relation to the selected polymer(s), selected plasticizer(s) and the applied amount of said polymer(s).

In preparation of tablets, either as monolithic drug containing cores for subsequent coating with a delayed release membrane or as a matrix for coated multiple units, additional ingredients may be needed to obtain suitable technical properties such as binders, disintegrants, bulk agents, glidants, lubricants, and coatings agents without effects on the drug release such as water soluble polymers, anti-tacking agents, colourants, pigments and waxes. Ingredients well known for such usage are for example described in "Handbook of pharmaceutical excipients", 2^{nd} edition, 1994, Pharmaceutical Press, London.

### Preparation of final dosage forms

Coated units may be filled into hard gelatine capsules or mixed with tablet excipients, such as fillers, binders, disintegrants, lubricants and other pharmaceutically acceptable additives, and be compressed into tablets. The compressed tablet is optionally covered with film-forming agents to obtain a smooth surface of the tablet and further enhance the mechanical stability of the tablet during packaging and transport. Such a tablet coat, which may be applied on a multiple unit tablet or a conventional tablet, may further comprise additives like anti-tacking agents, colourants and pigments or other additives to improve the tablet appearance.

Suitable drugs for the new formulations are IBAT inhibitor compounds such as described in the above-discussed documents, hereby incorporated by references.

The IBAT inhibitor compound could alternatively be a low permeability drug as defined in the Biopharmaceutical Classification System proposed by FDA.

A combination therapy according to the invention should preferably comprise simultaneously, separately or sequentially administration of an IBAT inhibitor compound and a bile acid binder. The IBAT inhibitor could preferably be formulated for ileum delivery and the bile acid binder could preferably be formulation for colon release.

### Medical and pharmaceutical use of the invention

The pharmaceutical formulations according to the present invention can be used in the treatment of hypercholesterolaemia. A suitable unit dose will vary with respect to the patients body weight, condition and disease severity. The dose will also depend on if it is to be used for prophylaxis or in the treatment of severe conditions, as well as the route of administration. The daily dose can be administered as a single dose or divided into two or more unit doses. An orally administered daily dose of an IBAT inhibitor is preferably within 0.1 - 1,000 mg, more preferable 1 - 100 mg.

A pharmaceutical formulation according to the present invention with a targeted delivery in the gastro intestinal tract provides a reduced systemic exposure, as can be measured by the area under the drug plasma concentration versus time curve (AUC), while maintaining or even increasing the therapeutic effect, as e.g. measured by serum cholesterol reduction.

A combination therapy comprising an IBAT inhibitor and a bile acid binder comprises preferably a low daily dose of the bile acid binder, such as less than 5 g of a resin, and more preferably less than 2 g. A dosage form with colon release of the bile acid binder could be constructed by any of the above described principles for delayed release formulations.

The following contemplated Examples are intended to illustrate, but in no way limit the scope of the invention.

### EXAMPLES

### Example 1

A formulation having the following composition can be prepared:

| | amount/capsule (mg) |
|---|---|
| IBAT inhibitor compound | |
| (1,5-benzothiazepine) | 10 |
| Non pareil spheres | 500 |
| Ethyl cellulose | 2 |
| Hydroxypropylmethyl cellulose | 10 |
| Eudragit L100-55 | 25 |
| Triethylcitrate | 2.4 |

The active drug can be dissolved together with ethyl cellulose and hydroxypropyl cellulose in ethanol 99 %. The mixture can then be sprayed onto the non-pareil spheres in a fluidized bed apparatus. Thereafter, the pellets can be dried and aerated to remove residual ethanol. The Eudragit L100-55 dispersion with addition of triethyl citrate can then be sprayed onto the drug beads in a fluidized bed apparatus. Subsequently, the coated beads can be filled in hard gelatine capsules after drying and sieving.

### Example 2

A formulation having the following composition can be prepared:

| | amount/tablet (mg) |
|---|---|
| IBAT inhibitor compound | |
| (1,5-benzothiazepine) | 10 |
| Silicon dioxide | 200 |
| Povidone K-25 | 20 |
| Eudragit FS30D | 30 |
| Microcrystalline cellulose | 250 |
| Sodium stearyl fumarate | 5 |

The active drug can be suspended in water and sprayed onto silicon dioxide cores of a predefined size in a fluidized bed apparatus. The drug pellets can be dried in an oven at 40° C for 24 h. Thereafter, a layer of Povidone K-25 can be applied on the beads from an ethanolic solution in a fluidized bed apparatus. A final coat of Eudragit FS30D dispersion can be applied thereafter in a fluidized bed. The coated beads can be mixed with microcrystalline cellulose and sodium stearyl fumarate in a mixer and subsequently compressed to tablets.

## Claims

1. An oral pharmaceutical formulation comprising an IBAT inhibitor compound and a bile acid binder, wherein the formulation is designed to deliver the bile acid binder in the colon.

2. The formulation according to claim 1, wherein the IBAT inhibitor compound is a low permeability drug as defined in the Biopharmaceutical Classification System FDA.

3. The pharmaceutical formulation according to either of claims 1 or 2 wherein the bile acid binder is a resin.

4. The pharmaceutical formulation according to claims 1-3, wherein the IBAT inhibitor is a benzothiazepine, particularly a 1,4-benzothiazepine or a 1,5-benzothiazepine.

5. The pharmaceutical formulation according to any one of claims 1-4, wherein the IBAT inhibitor and the bile acid binder are administered simultaneously, separately or sequentially.

6. The use of the pharmaceutical formulation according to any one of claims 1-5 in the manufacture of a medicament for use in the prophylactic or therapeutic treatment of hypercholesterolemia.

7. The use of a pharmaceutical formulation according to any one of claims 1-5 in the manufacture of a medicament for use in the prophylactic or therapeutic treatment of a subject suffering from, or susceptible to, diarrhoea during therapy comprising an IBAT inhibitor compound.

8. The use of a bile acid binder in the manufacture of a medicament for use in the treatment of diarrhoea during therapy comprising an IBAT inhibitor compound.

## Patentansprüche

1. Orale pharmazeutische Formulierung, enthaltend eine IBAT-Inhibitorverbindung und ein gallensäurebindendes Mittel, wobei die Formulierung auf die Verabreichung des gallensäurebindenden Mittels an das Colon zugeschnitten ist.

2. Formulierung nach Anspruch 1, wobei es sich bei der IBAT-Inhibitorverbindung um ein Arzneimittel mit geringer Permeabilität gemäß der Definition des Biopharmaceutical Classification Systems der FDA handelt.

3. Pharmazeutische Formulierung nach Anspruch 1 oder 2, wobei es sich bei dem gallensäurebindenden Mittel um ein Harz handelt.

4. Pharmazeutische Formulierung nach einem der Ansprüche 1-3, wobei es sich bei dem IBAT-Inhibitor um ein Benzothiazepin, insbesondere ein 1,4- Benzothiazepin oder ein 1,5-Benzothiazepin handelt.

5. Pharmazeutische Formulierung nach einem der Ansprüche 1-4, wobei der IBAT-Inhibitor und das gallensäurebindende Mittel gleichzeitig, getrennt voneinander oder nacheinander verabreicht werden.

6. Verwendung einer pharmazeutischen Formulierung nach einem der Ansprüche 1-5 zur Herstellung eines Medikaments zur Verwendung bei der profilaktischen oder therapeutischen Behandlung von Hypercholesterinamie.

7. Verwendung einer pharmazeutischen Formulierung nach einem der Ansprüche 1-5 zur Herstellung eines Medikaments zur Verwendung bei der profilaktischen oder therapeutischen Behandlung eines Patienten, der während einer Therapie mit einer IBAT-Inhibitorverbindung an Diarrhö leidet bzw. bei dem ein Risiko einer Diarrhö besteht.

8. Verwendung eines gallensäurebindenden Mittels zur Herstellung eines Medikaments zur Verwendung bei der Behandlung von Diarrhö während einer Therapie mit einer IBAT-Inhibitorverbindung.

## Revendications

1. Formulation pharmaceutique orale comprenant un composé inhibiteur du TABI et un liant d'acides biliaires, dans laquelle la formulation est conçue pour délivrer le liant d'acides biliaires dans le côlon.

2. Formulation selon la revendication 1, dans laquelle le composé inhibiteur du TABI est un médicament de faible perméabilité selon la définition du système de classification biopharmaceutique FDA.

3. Formulation pharmaceutique selon l'une ou l'autre des revendications 1 ou 2 dans laquelle le liant d'acides biliaires est une résine.

4. Formulation pharmaceutique selon les revendications 1-3, dans laquelle l'inhibiteur du TABI est une benzothiazépine, en particulier une 1,4-benzothiazépine ou une 1,5-benzothiazépine.

5. Formulation pharmaceutique selon l'une quelconque des revendications 1-4, dans laquelle l'inhibiteur du TABI et le liant d'acides biliaires sont administrés simultanément, séparément ou l'un après l'autre.

6. Utilisation de la formulation pharmaceutique selon l'une quelconque des revendications 1-5 dans la fabrication d'un médicament à utiliser dans le traitement prophylactique ou thérapeutique ou l'hypercholestérolémie.

7. Utilisation d'une formulation pharmaceutique selon l'une quelconque des revendications 1-5 dans la fabrication d'un médicament à utiliser dans le traitement prophylactique ou thérapeutique d'un sujet souffrant de, ou sujet à, la diarrhée pendant une thérapie comprenant un composé inhibiteur du TABI.

8. Utilisation d'un liant d'acides biliaires dans la fabrication d'un médicament à utiliser dans le traitement de la diarrhée pendant une thérapie comprenant un composé inhibiteur du TABI.
